Europäisches Patentamt

European Patent Office

Office européen des brevets

(19)

(11) Veröffentlichungsnummer : **0 136 440**
**B1**

(12) EUROPÄISCHE PATENTSCHRIFT

(45) Veröffentlichungstag der Patentschrift :
25.05.88

(51) Int. Cl.⁴ : **A 61 B 3/12**

(21) Anmeldenummer : 84108676.2

(22) Anmeldetag : 23.07.84

(54) Ophthalmologisches Gerät für die Untersuchung des Augenhintergrundes und Messvorrichtung für das Gerät.

(30) Priorität : 19.09.83 CH 5073/83

(43) Veröffentlichungstag der Anmeldung :
10.04.85 Patentblatt 85/15

(45) Bekanntmachung des Hinweises auf die Patenterteilung : 25.05.88 Patentblatt 88/21

(84) Benannte Vertragsstaaten :
AT BE DE FR GB IT LU NL SE

(56) Entgegenhaltungen :
DE-A- 2 423 105
DE-A- 2 645 340
DE-A- 3 117 699
FR-A- 2 069 843
FR-A- 2 178 629
GB-A- 2 058 343
GB-A- 2 112 171
US-A- 3 872 303

(73) Patentinhaber : Robert, Yves Dr.
Susenbergstrasse 24
CH-8044 Zürich (CH)

(72) Erfinder : Robert, Y. Dr.
Susenbergstrasse 24
CH-8044 Zürich (CH)
Erfinder : Hendrickson, Ph. Dr.
Geispelgasse 26 b
CH-4132 Muttenz (CH)

(74) Vertreter : Fillinger, Peter, Dr.
Rütistrasse 1a
CH-5400 Baden (CH)

**Beschreibung**

Die Erfindung betrifft eine fotometrische Messanordnung gemäss dem Oberbegriff des Patentanspruches 1 oder 8.

Bekannte Geräte für die Untersuchung des Augenhintergrundes sind die Spaltlampe und die Funduskamera. Die Spaltlampe dient zur visuellen Untersuchung des Augenhintergrundes und besteht aus einem mit einer Beleuchtungseinrichtung und einem Binokular ausgerüsteten Mikroskop. Das Mikroskop der Funduskamera hat zusätzlich einen Kameraansatz, damit der Augenhintergrund fotografisch untersucht werden kann. Für die Augenhintergrunddiagnostik hat man ferner Laserscanner verwendet, wobei der Augenhintergrund mit Hilfe eines kollimierten, durch das Auge fokussierten Laserstrahls punktweise abgetastet wurde (Fortschr. Ophtalmol. (1982) 79 ; 275 bis 277).

Mit den bekannten Geräten können zwar die verschiedenen Bereiche des Augenhintergrundes, insbesondere der Netzhaut, miteinander verglichen und so Anomalien einzelner Bereiche festgestellt werden ; es ist jedoch schwierig, die Langzeitentwicklung anomaler Bereiche zu beurteilen.

Aus der DE-A-3 117 699 ist ein Fluoro-Fotometer zur Bestimmung der Konzentration eines fluoreszenten Materials in einem Auge bekannt. Mittels einer Lichtquelle und einer geeigneten Optik wird ein Lichtstrahl durch die Pupille in das Auge gelenkt, um das fluoreszente Material, das zuvor dem Patienten in die Blutbahn gespritzt wurde, zur Fluoreszenz anzuregen. Dieses Gerät weist weiter eine Optik zur Abbildung des Weges und der Fluoreszenz in einer Abtastebene auf, wobei in der Abtastebene eine lineare Fotodiodenanordnung vorhanden ist. Die von der Fotodiodenanordnung gelieferten Daten werden in einem Prozessor erfasst und aufbereitet. Mit dieser Vorrichtung kann jedoch die Fluoreszenz am Augenhintergrund selbst, das heisst die Fluoreszenz von Sehnerv und Netzhaut selbst, nicht bestimmt werden, weil dort das Rauschen grösser als das Signal ist.

Der Erfindung liegt die Aufgabe zugrunde, ein Gerät der eingangs genannten Art mit einer Messvorrichtung auszurüsten, die es ermöglicht, zusätzlich zur visuellen Untersuchung des Augenhintergrundes in einfacher Weise den Absorptions- bzw. Reflexionsgrad bestimmter Stellen des Augenhintergrundes quantitativ zu bestimmen, damit die Langzeitentwicklung dieser Stellen beurteilt werden kann.

Die erfindungsgemässe Lösung dieser Aufgabe ist Gegenstand der Patentansprüche 1 und 8.

Mit dem erfindungsgemässen Gerät kann insbesondere der Reflexions- bzw. Absorptionsgrad des blinden Flecks, d. h. des Sehnervenkopfes (der Papille) von Zeit zu Zeit bestimmt werden. Die Erfindung geht dabei von der Erkenntnis aus, dass Aenderungen des Absorptions- bzw. Reflexionsgrades des blinden Flecks Anhaltspunkte für die Entstehung und Entwicklung eines Hirntumors oder des grünen Stars (Glaukoms) geben können.

Im folgenden wird anhand der Zeichnung ein Ausführungsbeispiel der Erfindung näher beschrieben. Es zeigen :

Fig. 1 eine schematische Seitenansicht eines ophthalmologischen Geräts, in Blickrichtung I in Fig. 2,

Fig. 2 eine schematische Draufsicht auf das Mikroskop des Geräts, in Blickrichtung II in Fig. 1 und

Fig. 3 eine gleiche Darstellung wie Fig. 1 mit einem monokularen ophthalmologischen Gerät mit Kameraanschluss.

Das Gerät besteht aus einer handelsüblichen Spaltlampe, die mit einer erfindungsgemässen Messvorrichtung ausgerüstet ist.

Die Spaltlampe hat eine Beleuchtungseinrichtung 1 mit einer Lampe 2 (Glühlampe bzw. Halogenlampe), einem Kollektor 3, einer verstellbaren Spaltblende 4 (Leuchtfeldblende) und einem Kondensor 5. Mittels eines (nicht dargestellten) Transformators kann die Beleuchtungsstärke der Lampe 2 variiert werden. Das aus dem Kondensor 5 austretende Lichtbündel wird von einem halbdurchlässigen Spiegel 7 auf das Patientenauge 8 gelenkt. Das Mikroskop 10 der Spaltlampe ist ein Binokularmikroskop, das zwei Objektive 11, 12 und zwei Okulartuben 13, 14 hat. Die optische Achse des Objektives 11 und Okulartubus 13 verläuft in einem spitzen Winkel von z. B. 13° zur optischen Achse des Objektives 12 und Okulartubus 14. Bei der handelsüblichen Spaltlampe sind in beide Okulartuben 13, 14 Okulare eingesetzt. Die beiden mittels der Okulare gesehenen Bilder unterscheiden sich ein wenig voneinander und ermöglichen so einen räumlichen Eindruck des Augenhintergrundes. Beim dargestellten Gerät ist in den Okulartubus 13 anstelle des Okulars ein okularförmiges Gehäuse 15 gesteckt, in dem eine Fotodiode 16 angeordnet ist, die zur weiter unten beschriebenen Messvorrichtung gehört. Durch die okularförmige Ausgestaltung des Gehäuses 15 ist dieses mit seiner zylindrischen Aussenfläche 150 genau in der zylindrischen Innenfläche des Okulartubus 13 längsachsial geführt, bis es mit dem Ringwulst 151 am rückseitigen Ende des Okulartubus 13 in Anschlag kommt. Der Abstand 152 der Ebene der Fotodiode 16 zur Anschlagebene des Ringwulstes 151 ist so gewählt, dass die Fotodiode 16 bei vollständig eingeschobenem Gehäuse in der Bildfeldebene 26 liegt. Im Okulartubus 14 sitzt das übliche Mikroskopokular 20, im vorliegenden Beispiel ein Huygenssches Okular, bei dem das reelle Bild zwischen der Feldlinse 21 und der Augenlinse 22 entsteht. In der strichpunktiert angedeuteten Bildfeldebene 23 ist eine Fadenkreuz-Platte 24 angeordnet.

Die lichtempfindliche Fläche der Fotodiode 16 ist in der Bildfeldebene 26 angeordnet, auf welche das Objektiv 11 den Augenhintergrund abbildet.

(Wegen der Feldlinse 21 ist der Abstand der Bildfeldebene 23 vom Objektiv 12 etwas kleiner als der Abstand der Bildfeldebene 26 vom Objektiv 11). Die lichtempfindliche Fläche der Fotodiode 16 ist kleiner als die bei der kleinsten Einstellung der Spaltblende 4 auf der Bildfeldebene 26 abgebildete Fläche, und sie ist genau an der Stelle der Bildfeldebene 26 angeordnet, die dem Fadenkreuz 24 in der Bildfeldebene 23 entspricht. Da die von den beiden Objektiven 11, 12 erzeugten Bilder nicht genau übereinstimmen und das Fadenkreuz 24 im Zentrum des Bildfeldes 23, d. h. in der Achse des Tubus 14 angeordnet ist, ist die lichtempfindliche Fläche der Fotodiode 16 entsprechend der Bildverschiebung gegenüber dem Zemtrum des Bildfelds 26 bzw. der Achse des Tubus 13 versetzt angeordnet.

Um sicherzustellen, dass die Fotodiode 16 beim Einsetzen des Gehäuses 15 in die richtige Lage gelangt, kann am Gehäuse 15 ein seitlich vorstehender Stift 153 vorgesehen sein, der in einen Längsschlitz des Tubus 13 einzuführen ist und der das Gehäuse 15 gegen ein Drehen gegenüber dem Tubus 13 sichert.

Die Anschlussleitung 28 der Fotodiode 16 ist an der Rückseite des okularförmigen Gehäuses 15 herausgeführt und an den einen Eingang eines Dividierers 29 angeschlossen, dessen anderer Eingang mit einer Fotodiode 30 verbunden ist, welche in der Beleuchtungseinrichtung 1 angeordnet ist und die Lichtintensität der Lampe 2 detektiert. Der Dividierer 29 ist Teil einer Anzeigevorrichtung 32, welche den durch den Dividierer gebildeten Quotienten der Lichtintensitäten der Fotodioden 30 und 16 digital an einer Anzeige 33 anzeigt. Der angezeigte Quotient ist ein Mass für den Absorptionsgrad der untersuchten Stelle des Augenhintergrundes, z. B. des blinden Flecks. Die Anzeigevorrichtung 32 kann zusätzlich einen Multiplizierer aufweisen, der den vom Dividierer 29 gebildeten Quotienten mit einem Faktor multipliziert, der z. B. bei der ersten Inbetriebnahme des Geräts so eingestellt wird, dass bei der weiter unten beschriebenen Messung des Absorptionsgrads des blinden Flecks eines gesunden Patentenauges der Wert 100 angezeigt wird.

Wie erwähnt ist die Leuchte 2 eine für die visuelle Untersuchung übliche Glühlampe, die polychromatisches Licht ausstrahlt. Damit der von der Anzeige 33 angezeigte Wert grundsätzlich dem durch die visuelle Untersuchung qualitativ abschätzbaren Absorptionsgrad der untersuchten Stelle des Augenhintergrunds entspricht, ist eine solche Fotodiode 16 gewählt, deren spektraler Empfindlichkeitsbereich dem Empfindlichkeitsbereich des menschlichen Auges angepasst ist oder ihn wenigstens umfasst. Am vorderen Ende des okularförmigen Gehäuses 15 kann dabei ein Halter für Farbfilter vorgesehen sein, damit zusätzlich die Absorption des Augenhintergrundes bei bestimmten Wellenlängenbereichen gemessen werden kann.

Das Gerät wird wie folgt verwendet : Zunächst wird mittels des Okulars 20 der von der Lampe beleuchtete Augenhintergrund visuell untersucht.

Dabei kann das okularförmige Gehäuse 15 aus dem Tubus 13 herausgezogen und ein zweites Okular eingesetzt werden, um einen räumlichen Eindruck zu erhalten. Für die Messung des Absorptions- bzw. Reflexionsgrads einer zu untersuchenen Stelle des Augenhintergrundes, insbesondere des blinden Flecks, wird das okularförmige Gehäuse 15 in den Tubus 13 eingesetzt, wobei der Stift des Gehäuses in den Längsschlitz des Tubus eingeführt und so sichergestellt wird, dass die Fotodiode 16 in die richtige Lage gelangt. Mit dem üblichen (nicht dargestellten) Einstellmechanismus bewegt der Arzt das Mikroskop 10, bis die zu untersuchende Stelle des Augenhintergrundes genau im Fadenkreuz 24 des Okulars 20 liegt. Der von der Anzeige 33 angezeigte Wert ist dann ein Mass für den Absorptionsgrad dieser Stelle des Augenhintergrundes.

Die Einstellung des Mikroskops für die Messung der gewünschten Stelle des Augenhintergrundes kann auch ohne Fadenkreuz 24 erfolgen, indem Länge und Breite der Spaltblende 4 fein eingestellt werden, bis nur noch die zu untersuchende Stelle beleuchtet ist, bzw. die zu untersuchende Stelle im Zentrum des beleuchteten Flecks liegt. Voraussetzung für dieses Untersuchungsverfahren ist selbstverständlich, dass die lichtempfindliche Fläche der Fotodiode 16 in der Achse des durch das Objektiv 11 einfallenden Strahlenbündels angeordnet ist.

Die Messvorrichtung mit dem Fotodioden 16, 30 und der Anzeigevorrichtung 32 kann für beliebige ophthalmologische Geräte der in der Einleitung genannten Art verwendet werden. Dabei ist ledigliche das Gehäuse 15 und die Fotodiodenanordnung an das jeweilige Gerät anzupassen. Bei einer mit Ramsdenschen Okularen ausgerüsteten Spaltlampe ist z. B. lediglich ein längerer Tubus des Gehäuses 15 erforderlich, damit die Fotodiode 16 in der Bildfeldebene angeordnet werden kann die vor der Feldlinse des Okulars liegt. Bei einer Funduskamera mit binokularem Okulartubus kann die Fotodiode 16, wie beim dargestellten Beispiel der Spaltlampe, in einem okularförmigen Gehäuse angeordnet werden. Sie kann aber auch in einem anstelle der Kamera am Kameraansatz zu befestigenden Gehäuse angeordnet werden und ist so auch für eine Funduskamera mit monokularem Okulartubus verwendbar.

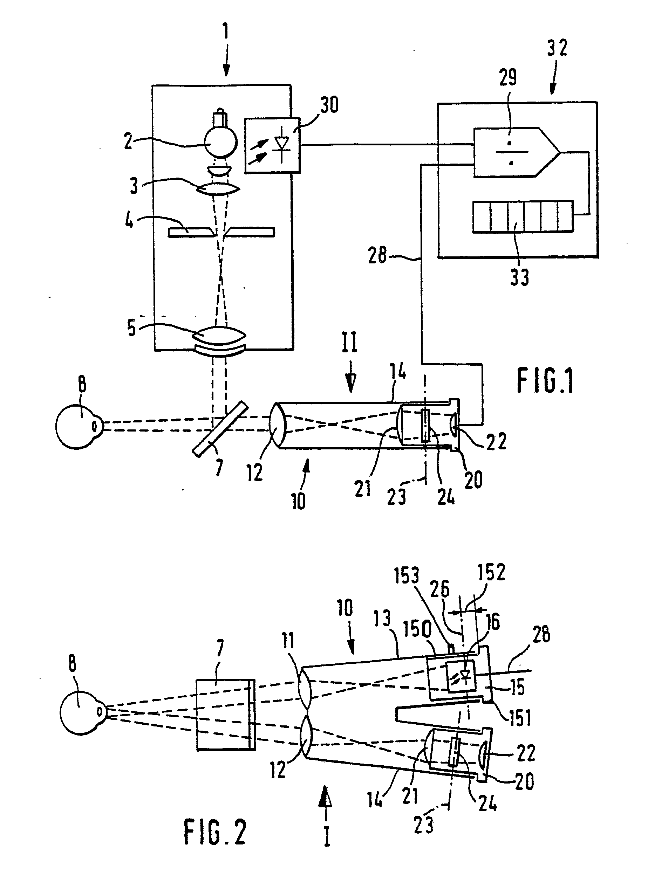

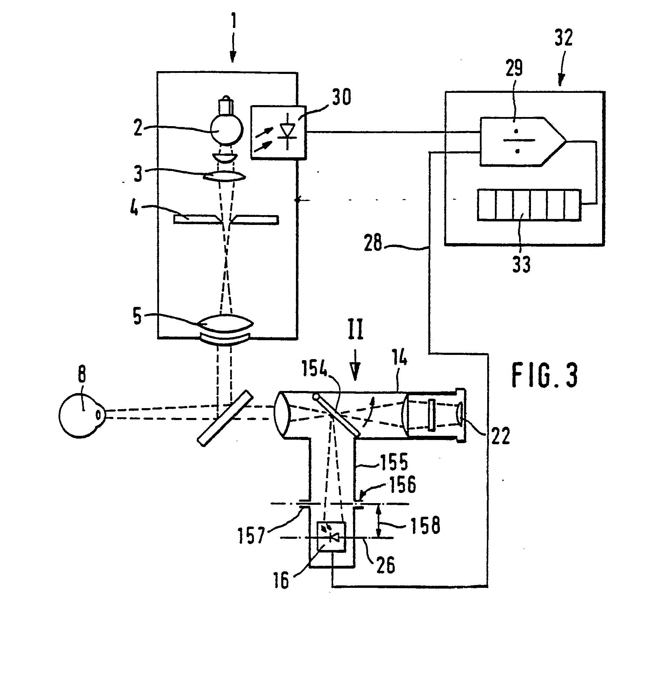

Fig. 3 zeigt eine der Fig. 1 entsprechende Darstellung einer monokularen Funduskamera. Die gleichen Teile sind mit den gleichen Hinweisziffern versehen, wie bei den Fig. 1 und 2, so dass auf deren wiederholende Beschreibung verzichtet wird. Im Tubus 13 ist ein Klappspiegel 154 angeordnet, der nach oben aus dem Strahlengang zur Augenlinse 22 verschwenkbar ist, so dass der Augenhintergrund durch die Augenlinse 22 beobachtet werden kann. Ist er nach unten in die in Fig. 3 gezeigte Stellung verschwenkt, wird das Licht in einen Kameraansatz 155 umgelenkt, der mit einem Bajonettverschluss 156 oder dgl. zum befestigen einer Kamera versehen ist. Das die Fotodiode 16 enthaltende Gehäuse 15 weist hier ein Komplementärteil 157 zum Bajonettverschluss 156 auf mit dem das Gehäuse 15 lagegerecht am Kameraanschluss 155 befestigbar ist. Der Abstand 158 der Fotodiode 16 zur Ebene des Bajonettverschlusses ist so gewählt, dass die Fotodiode 16 bei befestigtem Gehäuse 15 in der Bildebene 26 liegt.

Wegen des verhältnismässig grossen Kamerabildfeldes und der entsprechen geringen Leuchtdichte kann dabei für die Messung die zum Fotografieren vorgesehene Blitzlampe der Funduskamera verwendet werden. Die Fotodiode 30 wird in diesem Fall im Gehäuse der Blitzlampe angeordnet. Die Anzeigevorrichtung 32 kann dabei zwei Integrierer für die Integration der Ströme der beiden Fotodioden während der Blitzlichtdauer haben, wobei der Dividierer 29 den Quotienten der beiden Integralwerte bildet. Zur Vermeidung von Messfehlern infolge örtlich unregelmässigem Zündverhalten kann über die Blitzlampe eine Haube aus Opalglas gestülpt werden. Wegen der gerade für ein krankes Auge unerwünschten, u. U. sogar gefährlichen Blendung durch das Blitzlicht wird aber für die Messung mittels des erfindungsgemässen Geräts vorzugsweise das Licht der für die visuelle Untersuchung vorgesehenen Leuchte verwendet.

Die Fotodiode 30 könnte auch weggelassen werden, wenn gewährleistet ist, dass bei der Messung stets dieselbe Lampenspannung herrscht und die Lichtausbeute der Lampe konstant bleibt.

Anstelle oder zusätzlich zur Anzeigevorrichtung 32 könnte auch eine Aufzeichnungsvorrichtung vorgesehen sein, welche den vom Dividierer 29 gebildeten Quotienten bzw. das vom Multiplizierer gebildete Produkt aufzeichnet, z. B. ein Drucker oder ein Schreiber. Durch kontinuierliches Bewegen des Mikroskops kann mit dem Schreiber dann auch der Absorptionsverlauf längs, einer Linie des Augenhintergrunds aufgenommen werden.

Die erfindungsgemässe Lösung ermöglicht es dem Arzt, das von ihm für die visuelle Untersuchung des Augenhintergrunds bevorzugte, ophthalmologische Gerät, insbesondere eine Spaltlampe, unter Zuhilfenahme der Messvorrichtung für die Bestimmung des Absorptionsgrads einer bestimmten Stelle des Augenhintergrunds, z. B. des blinden Flecks, zu verwenden. Durch Vergleich der von Zeit zu Zeit bestimmten Absorptionswerte der in Frage stehenden Stelle des Augenhintergrunds, insbesondere des blinden Flecks, kann die Entstehung und der Verlauf von Krankheiten, z. B. eines Hirntumors oder des grünen Stars diagnostiziert werden.

**Patentansprüche**

1. Fotometrische Messvorrichtung für eine Spaltlampe, die eine Lichtquelle (2) und ein Stereomikroskop (10) aufweist, wobei der von der Lichtquelle beleuchtete Teil des Augenhintergrundes auf zwei Bildfeldern (23, 26) im Mikroskop abgebildet und durch je ein Okular (20) beobachtbar ist, oder für eine mit einem Stereomikroskop ausgerüstete Funduskamera, dadurch gekennzeichnet, dass die Messvorrichtung einen Fotodetektor (16) aufweist, der anstelle eines der beiden Okulare in einem der Bildfelder (26) angeordnet wird und die Intensität des vom Augenhintergrund reflektierten Lichts an der durch das andere Okular (20) beobachteten Stelle misst.

2. Messvorrichtung nach Anspruch 1, dadurch gekennzeichnet, dass sie einen zweiten Fotodetektor (30), der die Lichtintensität der Leuchte (2) oder einer zusätzlichen im Gerät vorgesehenen Blitzlampe misst, und einen Dividierer (29) hat, der den Quotienten aus den von den beiden Fotodetektoren (16, 30) gelieferten Signalen bildet.

3. Messvorrichtung nach Anspruch 1 oder 2, bei der wenigstens annähernd im Zentrum des ersten Bildfeldes (23) eine Markierung (24), beispielsweise ein Fadenkreuz, angeordnet ist, dadurch gekennzeichnet, dass die lichtempflindliche Fläche des Fotodetektors (16) klein gegenüber der Bildfläche ist und genau an der Stelle im zweiten Bildfeld (26) angeordnet ist, die der Markierung (24) im ersten Bildfeld (23) entspricht.

4. Messvorrichtung nach Anspruch 1 oder 2 mit einer verstellbaren Leuchtfeldblende (4), insbesondere einer Spaltblende, mittels der die Grösse der von der Leuchte (2) am Augenhintergrund ausgeleuchteten Fläche einstellbar ist, dadurch gekennzeichnet, dass die lichtempfindliche Fläche des Fotodetektors (16) kleiner als die bei der kleinsten Einstellung der Leuchtfeldblende (4) auf dem zweiten Bildfeld (26) abgebildete Fläche und in deren Zentrum angeordnet ist.

5. Messvorrichtung nach einem der Ansprüche 1 bis 4, bei der die Leuchte (2) polychromatisches Licht ausstrahlt, dadurch gekennzeichnet, dass der spektrale Empfindlichkeitsbereich des ersten Fotodetektors (16) demjenigen des menschlichen Auges angepasst ist oder ihn wenigstens umfasst.

6. Messvorrichtung nach einem der Ansprüche 1 bis 5, dadurch gekennzeichnet, dass der Fotodetektor eine Fotodiode (16) ist.

7. Messvorrichtung nach einem der Ansprüche 1 bis 6, dadurch gekennzeichnet, dass der Fotodetektor (16) in einem okularförmigen Gehäuse (15) angeordnet ist, das auswechselbar in einem der Okulartuben (13) angeordnet werden kann.

8. Fotometrische Messvorrichtung für eine Funduskamera, die eine Lichtquelle (2) und einen monokularen Beobachtungsteil (14) mit einem Okular (22) sowie einen Klappspiegel (154) und einen in bezug auf den Klappspiegel konjugiert zum Okular (22) angeordneten Kameraansatz (155) aufweist, oder für eine mit einem Kameraansatz ausgerüstete Spaltlampe, dadurch gekennzeichnet, dass die Messvorrichtung einen Fotodetektor (16) aufweist der anstelle einer Kamera am Kameraansatz (155) angeordnet wird und die Intensität des vom Augenhintergrund reflektierten Lichts an der bei anderer Stellung des Klappspiegels (154) durch das Okular (22) beobachteten Stelle misst.

9. Messvorrichtung nach Anspruch 9, dadurch gekennzeichnet, dass sie einen zweiten Fotodetektor (30) zum Messen der Lichtintensität der Lichtquelle (2) aufweist, und dass ein Dividierer (29) vorhanden ist, der den Quotienten aus den von den beiden Fotodetektoren (16, 30) gelieferten Signalen bildet.

10. Messvorrichtung nach Anspruch 8 oder 9, dadurch gekennzeichnet, dass die lichtempfindliche Fläche des Fotodetektors (16) klein gegenüber der Bildfeldfläche ist.

11. Messvorrichtung nach Anspruch 8 oder 9, dadurch gekennzeichnet, dass der spektrale Empfindlichkeitsbereich des Fotodetektors (16) demjenigen des menschlichen Auges angepasst ist oder ihn wenigstens umfasst.

**Claims**

1. Photometric measuring device for a slit lamp, which comprises a light source (2) and a stereomicroscope (10), that portion of the eye fundus which is illuminated by the light source being represented on two image fields (23, 26) in the microscope and being observable through respective eyepieces (20), or for a fundus camera equipped with a stereomicroscope, characterised in that the measuring device comprises a photodetector (16) which is arranged in place of one of the two eyepieces in one of the image fields (26) and measures the intensity of the light reflected by the eye fundus at the place observed by the other eyepiece (20).

2. Measuring device according to claim 1, characterised in that it has a second photodetector (30), which measures the light intensity of the lamp (2) or of an additional flashbulb provided in the apparatus, and also a divider (29) which forms the quotient from the signals delivered by the two photodetectors (16, 30).

3. Measuring device according to claim 1 or 2, wherein a marking (24), for example a reticle, is arranged at least approximately in the centre of the first image field (23), characterised in that the light-sensitive surface of the photodetector (16) is small relatively to the image area and is arranged precisely at the place in the second image field (26) which corresponds to the marking (24) in the first image field (23).

4. Measuring device according to claim 1 or 2, with an adjustable radiant field diaphragm (4), especially a slit diaphragm, by means of which the size of the area illuminated by the lamp (2) on the eye fundus is adjustable, characterised in that the light-sensitive surface of the photodetector (16) is smaller than the area represented on the second image field (26) at the smallest setting of the radiant field diaphragm (4).

5. Measuring device according to one of claims 1 to 4, wherein the lamp (2) radiates polychromatic light, characterised in that the spectral sensitivity range of the first photodetector (16) is adapted to that of the human eye or at least comprehends or includes it.

6. Measuring device according to one of claims 1 to 5, characterised in that the photodetector is a photodiode (16).

7. Measuring device according to one of claims 1 to 6, characterised in that the photodetector (16) is arranged in an eyepiece-shaped housing (15) which can be arranged interchangeably in one of the eyepiece tubes (13).

8. Photometric measuring device for a fundus camera, which comprises a light source (2) and a monocular observation part (14) with an eyepiece (22) and also a pivotably movable mirror (154) and a camera extension (155) arranged conjugately to the eyepiece (22) relatively to the pivotable mirror, or for a slit lamp equipped with a camera extension, characterised in that the measuring device comprises a photodetector (16) which is arranged in place of a camera on the camera extension (155) and measures the intensity of the light reflected by the eye fundus at the place observed through the eyepiece (22) with another position of the pivotable mirror (154).

9. Measuring device according to claim 9, characterised in that it comprises a second photodetector (30) for measuring the light intensity of the light source (2), and that a divider (29) is provided which forms the quotient from the signals delivered by the two photodetectors (16, 30).

10. Measuring device according to claim 8 or 9, characterised in that the light-sensitive surface of the photodetector (16) is small relatively to the image field area.

11. Measuring device according to claim 8 or 9, characterised in that the spectral sensitivity range of the photodetector (16) is adapted to that of the human eye or at least includes it.

**Revendications**

1. Dispositif de mesure photométrique, pour une lampe à fente, pourvue d'une source de lumière (2) et d'un microscope stéréoscopique (10), la partie du fond d'œil qui est éclairée par la source de lumière étant à cette occasion observable sur deux champs d'images (23, 26) dans le microscope et à travers un oculaire (20), ou bien pour un appareil pour la photographie du fond de l'œil équipée d'un microscope stéréoscopique, caractérisé en ce que le dispositif de mesure est pourvu d'un photodétecteur (16), disposé à la place d'un des deux oculaires, dans un des champs d'images (26) et qui mesure l'intensité de la lumière réfléchie par le fond d'œil, à l'endroit qui est observé par l'autre oculaire (20).

2. Dispositif de mesure selon la revendication 1, caractérisé en ce qu'il possède un deuxième photodétecteur (30), qui mesure l'intensité lumineuse du luminaire (2) ou bien d'une lampe à éclair prévue supplémentairement dans l'appareil, et possède un diviseur (29) qui forme le quotient des signaux délivrés par les deux photodétecteurs (16, 30).

3. Dispositif de mesure selon la revendication 1 ou 2, dans lequel au moins un marquage (24), par exemple un réticule à croisée est disposé approximativement au centre du premier champ d'image (23), caractérisé en ce que la surface du photodétecteur (16) qui est sensible à la lumière est petite par rapport à la surface d'image et est disposée dans le deuxième champ d'image (26), exactement à l'endroit qui correspond au marquage (24), dans le premier champ d'image (23).

4. Dispositif de mesure selon la revendication 1 ou 2, avec un diaphragme de champ de lumière (4), en particulier un diaphragme à fente, au milieu duquel la grandeur de la surface éclairée sur le fond de l'oeil par la lampe (2) est réglable, caractérisé en ce que la surface du photodétecteur (16) qui est sensible à la lumière est plus petite que la surface formée, lors du plus petit réglage du diaphragme de champ de lumière (4), sur le deuxième champ d'image (26) et est disposée en son centre.

5. Dispositif de mesure selon l'une des revendications 1 à 4, dans lequel la lampe (2) rayonne une lumière polychromatique, caractérisé en ce que la zone de sensibilité spectrale du premier photodétecteur (16) est adaptée à celle de l'oeil humain ou bien au moins l'englobe.

6. Dispositif de mesure selon l'une des revendications 1 à 5, caractérisé en ce que le photodétecteur est une photodiode (16).

7. Dispositif de mesure selon l'une des revendications 1 à 6, caractérisé en ce que le photodétecteur est disposé dans un carter (15) de forme oculaire, qui peut être disposé de manière interchangeable dans l'un des tubes oculaires (13).

8. Dispositif de mesure photométrique pour un appareil pour la photographie du fond de l'oeil, qui possède une source de lumière (2) et une partie d'observation (14) monoculaire avec un oculaire (22) ainsi qu'un miroir basculant (154) et un appendice de chambre (155) disposé de manière conjuguée sur l'oculaire (22), en relation avec le miroir basculant, ou bien pour une lampe à fente équipée d'un appendice de chambre, caractérisé en ce que le dispositif de mesure possède un photodétecteur (16) qui est disposé à la place d'une chambre sur l'appendice de chambre (155) et qui mesure l'intensité de la lumière réfléchie par le fond de l'oeil à l'endroit qui est observé à travers l'oculaire (22), dans une autre position du miroir basculant (154).

9. Dispositif de mesure selon la revendication 9, caractérisé en ce qu'il est pourvu d'un deuxième photodétecteur (30) pour mesurer l'intensité lumineuse de la source lumineuse (2), comprenant un diviseur (29), qui forme le quotient entre les signaux délivrés par les deux photodétecteurs (16, 30).

10. Dispositif de mesure selon la revendication 8 ou 9, caractérisé en ce que la surface sensible à la lumière du photodétecteur (16) est petite par rapport à la surface de champ d'image.

11. Dispositif de mesure selon la revendication 8 ou 9, caractérisé en ce que le champ de sensibilité spectrale du photodétecteur (16) est adapté à celui de l'oeil humain ou bien au moins l'englobe.

FIG.1

FIG.2

FIG. 3